# EUROPEAN PATENT APPLICATION

(11) **EP 4 502 181 A1**
(43) Date of publication of application: **05.02.2025**
(21) Application number: 23781406.6
(22) Date of filing: 30.03.2023
(51) Int. Cl.: C12Q 1/6883

(54) **IFI16 MUTANT GENE AS MARKER FOR PREDICTING, DIAGNOSING, OR PROGNOSING CHRONIC LIVER DISEASE AND USE THEREOF**

(30) Priority: 30.03.2022 KR 20220039809
(71) Applicant: National Cancer Center, Goyang-si, Gyeonggi-do 10408 (KR); Ajou University Industry-Academic Cooperation Foundation, Suwon-si, Gyeonggi-do 16499 (KR); Seoul National University R&DB Foundation, Seoul 08826 (KR); Dong-A University Research Foundation for Industry-Academy Cooperation, Busan 49315 (KR); ON Hospital, Busan 47261 (KR)
(72) Inventor: LEE, Yeon Su, Gimpo-si, Gyeonggi-do 10085 (KR); PARK, Gaeul, Goyang-si, Gyeonggi-do 10445 (KR); LEE, Yong Sun, Goyang-si, Gyeonggi-do 10416 (KR); WOO, Hyun Goo, Suwon-si, Gyeonggi-do 16505 (KR); KIM, Do Yoon, Suwon-si, Gyeonggi-do 16239 (KR); SEONG, Rho Hyun, Seongnam-si, Gyeonggi-do 13589 (KR); BAEK, Yang Hyun, Busan 49201 (KR); HAN, Sang Young, Sacheon-si, Gyeongsangnam-do 52543 (KR)
(74) Representative: Dehns
(86) International application number: PCT/KR2023/004303
(87) International publication number: WO 2023/191555

(57) **Abstract**

The present invention relates to an Interferon Gamma Inducible Protein 16 (IFI16) mutant gene and its use as a marker for predicting, diagnosing, or prognosticating risk or severity of chronic liver disease.

In the present invention, as a result of performing genomic analysis on NAFLD and NASH patient groups, it was confirmed that the frequency of IFI16 single-nucleotide variants (SNVs) including rs2276404, rs73021847, rs7532207, and rs6940 was increased, and the expression of the IFI16 mutant gene was increased depending on the disease stage of liver disease. Furthermore, we confirmed that the IFI16 SNV was highly expressed in infiltrating macrophages, playing a role in macrophage-induced inflammatory processes, and that the IFI16 variant bound more strongly to dsDNA than wild-type IFI16, exacerbating the impaired mitochondrial DNA-sensing response signaling of the IFI16-PYCARD-CASP1 pathway. Thus, the IFI16 mutant gene of the present invention may be useful for predicting, diagnosing, or prognosticating risk or severity of chronic liver disease.

## Description

### [Technical field]

The present invention relates to an Interferon Gamma Inducible Protein 16 (IFI16) mutant gene as a marker for predicting, diagnosing, or prognosticating risk or severity of chronic liver disease and uses thereof, and more particularly to a biomarker composition for predicting, diagnosing, or prognosticating risk or severity of chronic liver disease comprising the IFI16 mutant gene, and to a composition for predicting, diagnosing, or prognosticating risk or severity of chronic liver disease using the biomarker, kits, and methods for providing information for predicting, diagnosing, or prognosticating risk or severity of chronic liver disease.

### [Background art]

The socioeconomic burden of chronic liver disease in Korea was approximately KRW 3.7 trillion in 2010, making it the most serious disease in the country. Liver cancer and liver disease have the highest mortality rates in Korea, especially among those in their 40s and 50s.

Non-alcoholic fatty liver disease (NAFLD), one of the chronic liver diseases, is a progressive liver disease that ranges from simple steatosis to non-alcoholic steatohepatitis (NASH). In particular, non-alcoholic steatohepatitis (NASH) is a progressive disease of the liver characterized by fatty acid accumulation, hepatocyte injury, and inflammation that histologically resembles alcoholic hepatitis, and is a major step in the process of progression from hepatic steatosis to cirrhosis and liver failure. The incidence of NASH has been increasing in recent years, and patients progressing to NASH are experiencing increasing liver-related morbidity and mortality.

Histologic examination of liver biopsy specimens is the standard method for diagnosing the activity, stage, or severity of chronic liver disease, including NASH, but liver biopsy is invasive. In addition, there are limitations to performing biopsies on all of the ever-increasing number of patients with liver disease, and liver biopsies have side effects that can include pain, bleeding, and in rare cases, death (Rana L Smalling et al., Am J Physiol Gastrointest Liver Physiol., 305(5): G364-74, 2013; Korean Public Patent No. 10-2020-0051676).

For early diagnosis of liver disease or prediction of progression to chronic liver disease, methods have been developed to diagnose liver disease by analyzing the expression patterns of marker genes using microarray methods to identify relevant genes that can serve as predictive or diagnostic markers, including unsupervised clustering algorithms and supervised algorithmic methods. While unsupervised clustering analysis is very useful for extracting the intrinsic biological meaning of a sample, it is difficult to provide statistical accuracy of the results, and it is difficult to control the number of genes being measured appropriately. In addition, the probability of predicting the onset of liver disease is not accurate with these conventional methods, and in the case of genes that can be predictive or diagnostic markers, the signaling system involved in the development of liver disease in a cell is not regulated by a single gene but by a complex of genes, so diagnosing liver disease by analyzing the expression patterns of specific genes is also inaccurate.

Therefore, there is a need to develop new methods to more accurately and easily predict and diagnose the likelihood of developing chronic liver disease.

### [Disclosure]

### [Technical Problem]

Therefore, in an effort to develop a more effective biomarker for the prediction, diagnosis, or prognosis of chronic liver disease risk or severity, the present inventors performed an integrated genomic/transcriptomic analysis on a group of NAFLD and NASH patients and identified that the expression of the IFI16 (Interferon Gamma Inducible Protein 16) mutant (single-nucleotide variant: SNV) gene was increased with the stage of liver disease.

Accordingly, it is an object of the present invention to provide a biomarker composition for predicting, diagnosing, or prognosticating risk or severity of chronic liver disease comprising the IFI16 (Interferon Gamma Inducible Protein 16) mutant gene.

Another object of the present invention is to provide a composition for predicting, diagnosing, or prognosticating risk or severity of chronic liver disease, and a kit for predicting, diagnosing, or prognosticating risk or severity of chronic liver disease comprising an agent capable of detecting the IFI16 mutant gene.

Another object of the present invention is to provide a method of providing information for predicting, diagnosing, or prognosticating risk or severity of chronic liver disease using the IFI16 mutant gene.

### [Technical solution]

To fulfill the purposes described above, the present invention provides a biomarker composition for predicting, diagnosing, or prognosticating risk or severity of chronic liver disease, comprising an IFI16 (Interferon Gamma Inducible Protein 16) mutant gene or an IFI16 mutant protein.

In a preferred embodiment of the present invention, the IFI16 mutant gene may be one or more single-nucleotide variants (SNVs) selected from the group consisting of rs2276404, rs73021847, rs7532207, and rs6940 of the IFI16 gene, and the IFI16 mutant protein may be a missense variant (T723S) in which a threonine is replaced by a serine at position 723 of the amino acid sequence consisting of SEQ ID NO: 14.

In another preferred embodiment of the present invention, the chronic liver disease may be non-alcoholic fatty liver disease (NAFLD) or non-alcoholic steatohepatitis (NASH).

To fulfill other purposes, the present invention provides a composition for predicting, diagnosing, or prognosticating risk or severity of chronic liver disease, comprising a detection agent for the IFI16 mutant gene or IFI16 mutant protein.

The present invention also provides a kit for predicting, diagnosing, or prognosticating risk or severity of chronic liver disease, comprising a detection agent for the IFI16 mutant gene or IFI16 mutant protein.

In a preferred embodiment of the present invention, the detection agent may be a primer pair, probe, or antisense nucleotide that specifically binds to the mutant gene, or an antibody, interacting protein, ligand, nanoparticle, or aptamer that specifically binds to a mutant protein.

To fulfill another purpose, the present invention also provides a method of providing information for predicting, diagnosing, or prognosticating risk or severity of chronic liver disease, comprising:
(a) the step of extracting genomic DNA from a biological sample of the patient; and
(b) the step of detecting an IFI16 mutant gene or an IFI16 mutant protein in the extracted genomic DNA.

In a preferred embodiment of the invention, the IFI16 mutant gene may be one or more single-nucleotide variants (SNVs) selected from the group consisting of rs2276404, rs73021847, rs7532207, and rs6940 of the IFI16 gene, and the IFI16 mutant protein may be a missense variant (T723 S) in which a threonine is replaced by a serine at position 723 of the amino acid sequence consisting of SEQ ID NO: 14.

In another preferred embodiment of the present invention, the method of providing information may provide information that there is a high risk or severity of progression to chronic liver disease, chronic liver disease occurs, or the prognosis for chronic liver disease is poor when the IFI16 mutant gene or IFI16 mutant protein is detected or increased in expression.

In another preferred embodiment of the present invention, the chronic liver disease may be non-alcoholic fatty liver disease (NAFLD) or non-alcoholic steatohepatitis (NASH).

### [Advantageous Effects]

In the present invention, as a result of performing genomic analysis on NAFLD and NASH patient groups, it was confirmed that the frequency of IFI16 single-nucleotide variants (SNVs) including rs2276404, rs73021847, rs7532207, and rs6940 was increased, and the expression of the IFI16 mutant gene was increased depending on the disease stage of liver disease. Furthermore, we confirmed that the IFI16 SNV was highly expressed in infiltrating macrophages, playing a role in macrophage-induced inflammatory processes, and that the IFI16 variant bound more strongly to dsDNA than wild-type IFI16, exacerbating the impaired mitochondrial DNA-sensing response signaling of the IFI16-PYCARD-CASP1 pathway. Thus, the IFI16 mutant gene of the present invention can be useful for predicting, diagnosing, or prognosticating risk or severity of chronic liver disease.

### [Description of Drawings]

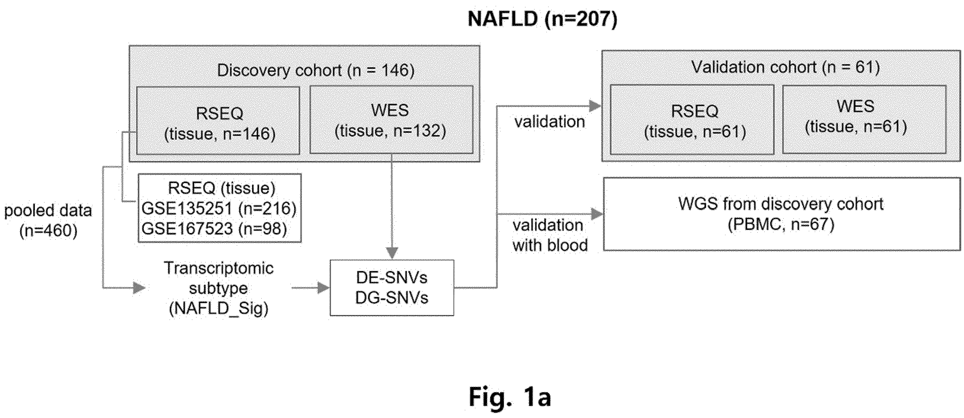

Figure 1A is a schematic diagram showing the biomarker selection process for diagnosing chronic liver disease.

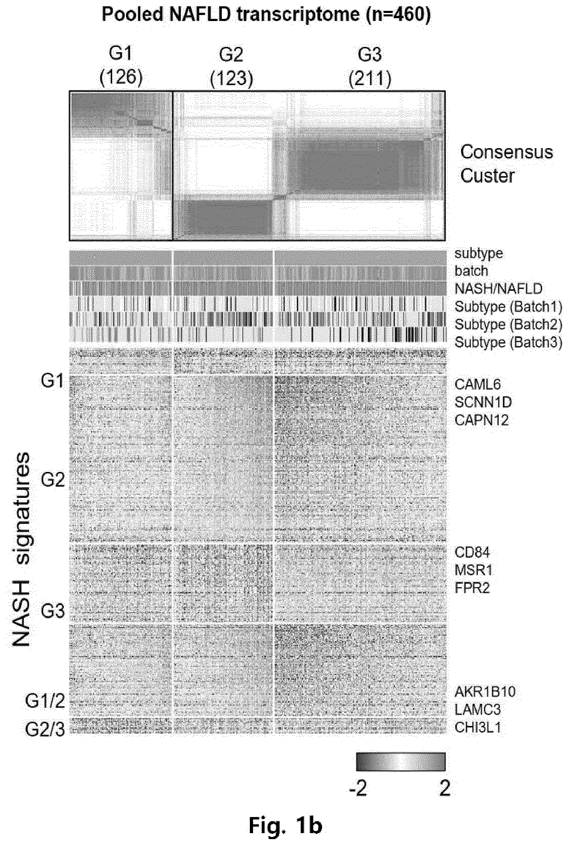

Figure 1B is a plot showing the analysis of expression patterns according to class after classifying classes (G1 to G3, subtype) into consensus clusters to select differentially expressed genes (DEGs) from integrated NAFLD transcriptome data.

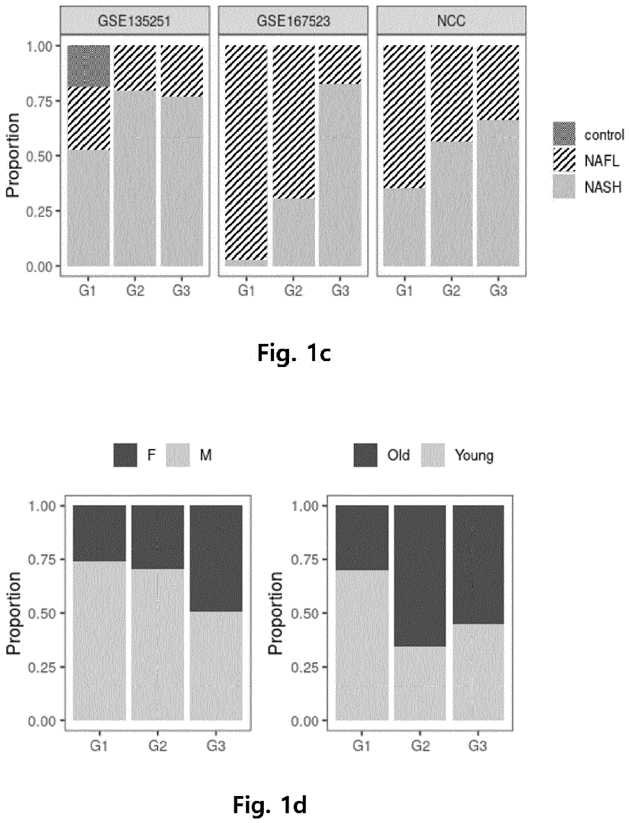

Figure 1C is a plot analyzing the proportion of chronic liver disease stages (right) according to each analysis group according to the classes classified in Figure 1B.
Figure 1D is a plot analyzing the gender ratio (left) and age ratio (right) according to the classes classified in Figure 1B.

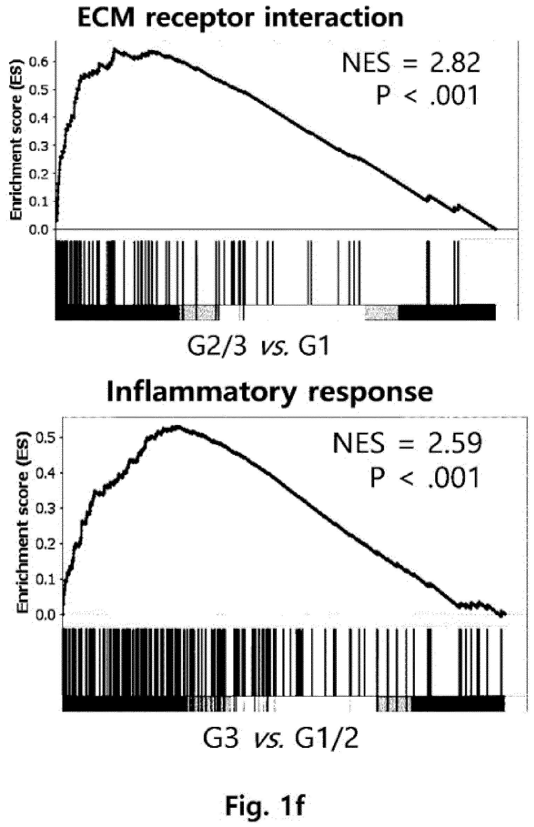

Figure 1F is a plot analyzing the degree of fibrosis according to the classes classified in Figure 1B by dividing them into the NCC analysis group (left) and the GSE135251 analysis group (right).
Figure 1E is a plot comparing and analyzing the enrichment score according to class classification using RNA-seq data (n=460) divided into classes in Figure 1B.
Figure 2A is a schematic diagram showing the process of selecting genes with single-nucleotide variant (SNV) through whole exome sequencing (WES) in a group of NCC patients.
Figure 2B is a plot analyzing the mutation rate of genes selected through the process of Figure 2A by class (White: Missing (Low depth), Gray: WT, Green: MUT).
Figure 2C is a plot analyzing the expression pattern of the IFI16 gene by class using GSE135251, GSE167523, and the National Cancer Center (NCC) RNA-seq (n=460) dataset.
Figure 2D is a plot analyzing the expression pattern of the IFI16 gene according to the IFI16 rs6940 SNV genotype.
Figure 2E top is a plot of the variation patterns of four DE-DSNVs in the IFI16 gene, rs2276404 (Promoter), rs73021847 (Enhancer), rs7532207 (Enhancer), and rs6940 (Missesnse variants), when whole genome analysis (WGS) was performed on peripheral blood mononuclear cells (PBMCs) from a group of NCC patients.

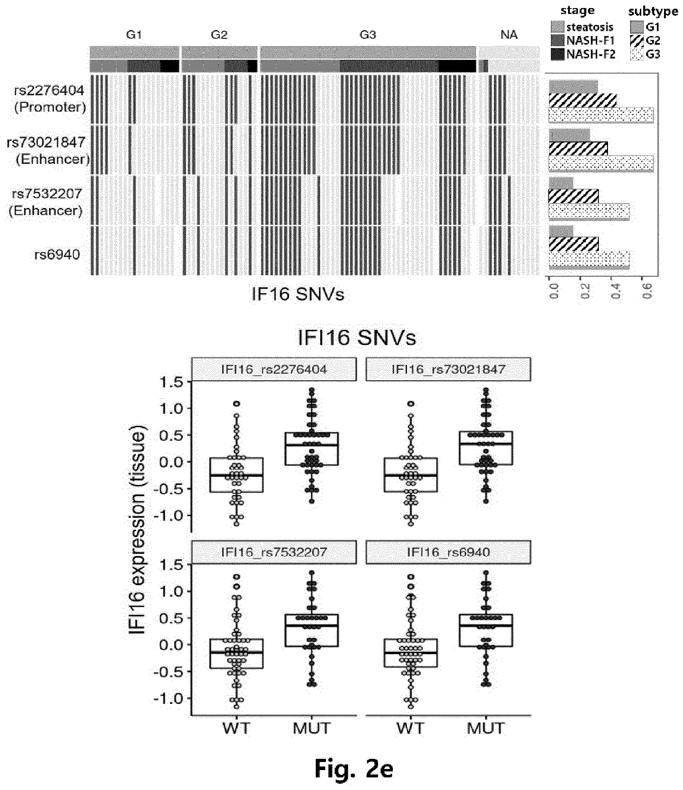

Figure 2E bottom analyzes the difference in expression values according to the genotype of the 4 SNVs (WT vs Mut) using RNA-seq expression values of the 4 SNVs and matched patients.

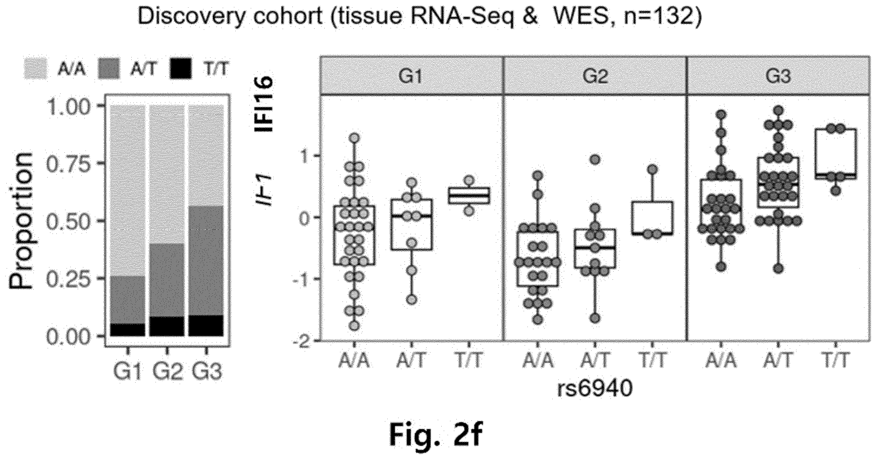

Figure 2F shows a class-wise analysis of the expression of the IFI16 SNV, rs6940 in the wild type (A/A), heterozygous mutant (A/T) and homozygous mutant (T/T).

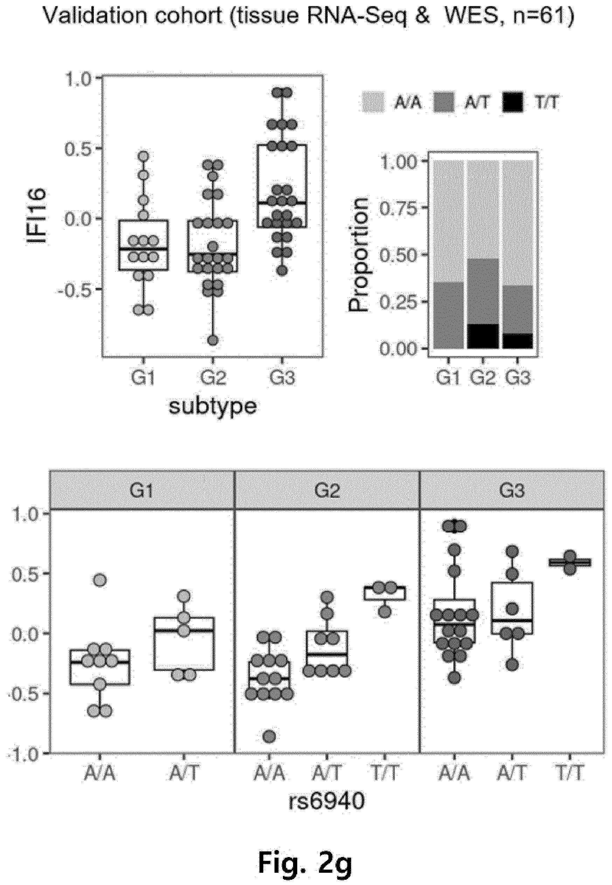

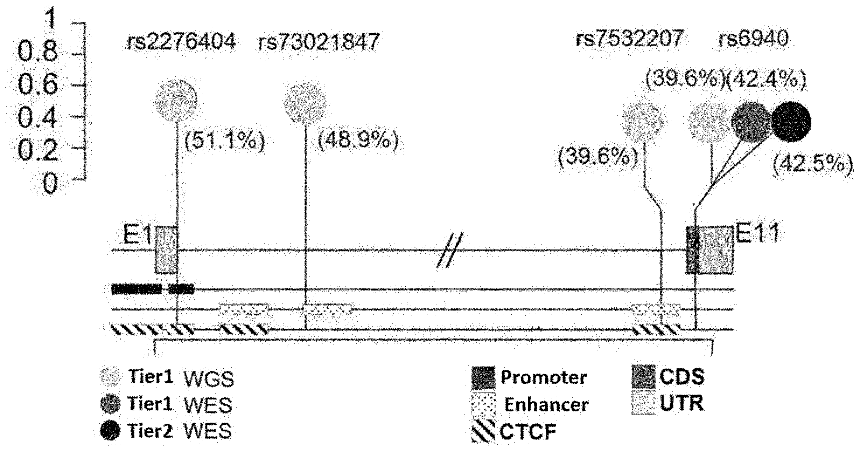

Figure 2G shows the expression pattern of the IFI16 gene analyzed by class (top) and the degree of expression of the IFI16 SNV, rs6940 in the wild type (A/A), heterozygous mutant (A/T), and homozygous mutant (T/T) analyzed by class (bottom), in the validation set.
Figure 2H is a Lolipop plot for the IFI16 SNV.

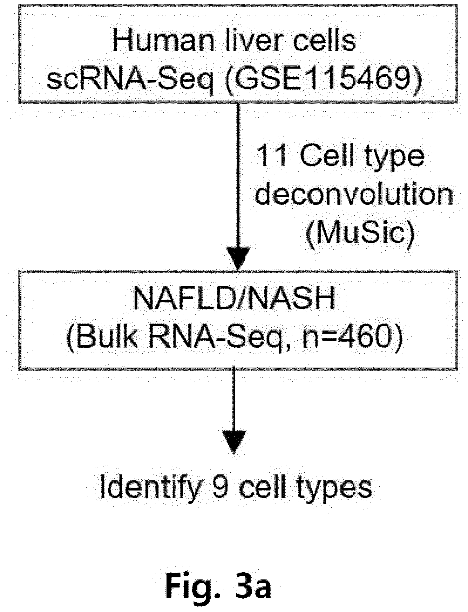

Figure 3A is a schematic diagram of the process of NAFLD/NASH-specific cell type selection by single-cell RNA sequencing (scRNA-Seq) of human hepatocytes.

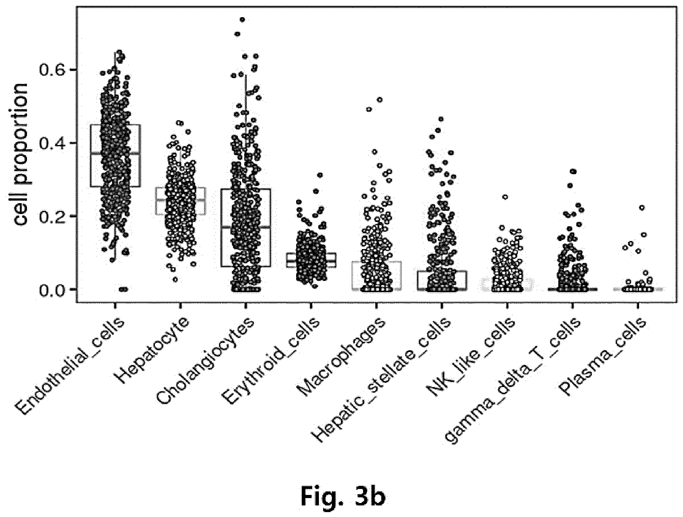

Figure 3B is a plot illustrating the extent of cell abundance according to the cell types selected in Figure 3A.

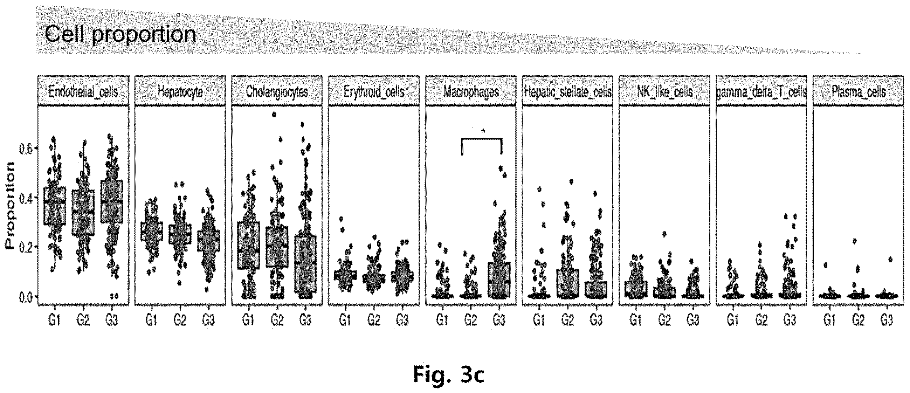

Figure 3C shows the proliferation of each cell type analyzed by the classes in Figure 1B.

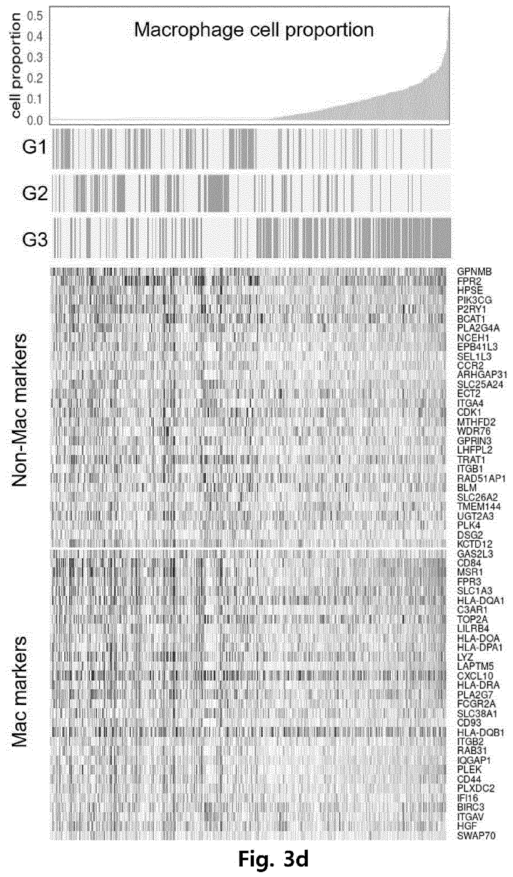

Figure 3D shows the expression patterns of genes that correlate with macrophage proportion and gene expression, divided into macrophage markers and non-macrophage markers, by analyzing the correlation of macrophage and gene expression patterns in the pooled RSEQ data (n=460) of Figure 1B.

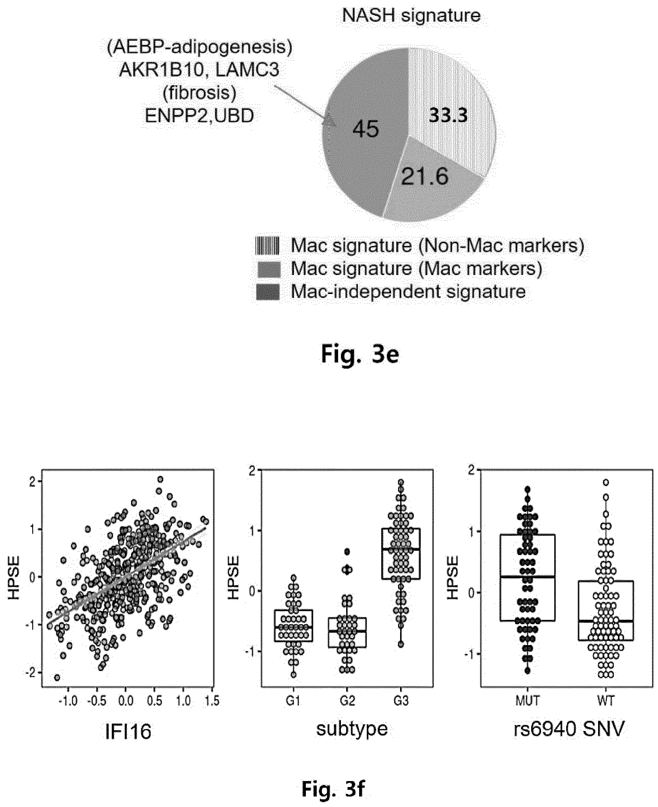

Figure 3E shows the differentially expressed genes by class, categorized into three types: macrophage signatures, Marker/Non-markers, and Mac-independent signatures, using the class-specific differentially expressed genes (DEGs) from FIG. 1B and the macrophage-associated gene data from FIG. 3D.
Figure 3F is a plot of the correlation between IFI16 gene and HPSE gene expression (left), HPSE gene expression by class (middle), and HPSE gene expression with and without IFI16 rs6940 mutation (right), using the pooled RSEQ data (n=460) from Figure 1B.

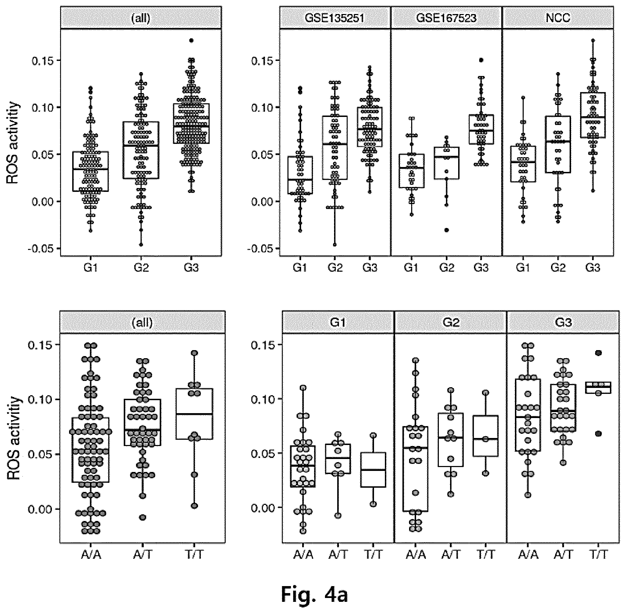

Figure 4A shows the expression of mitochondrial-related genes and ROS activity during NAFLD progression analyzed by class (top) and IFI16 rs6940 genotype (bottom).

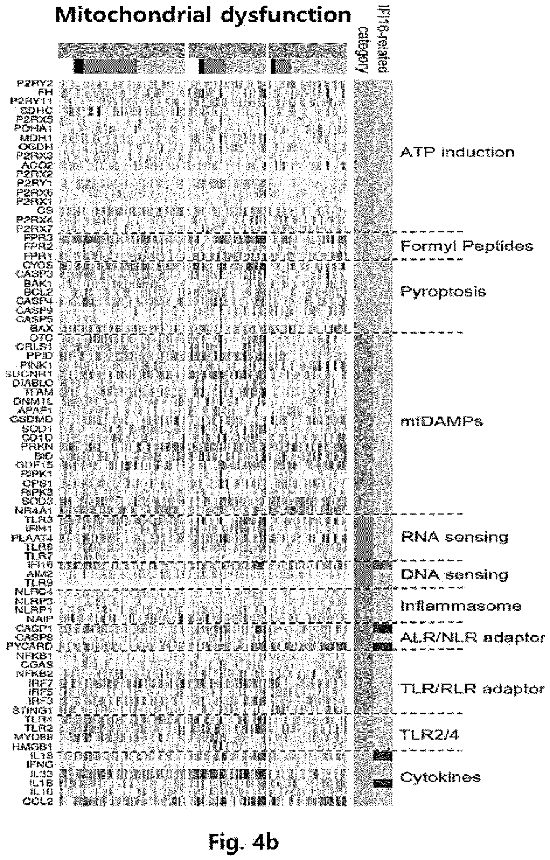

Figure 4B shows an analysis of the expression patterns of genes related to mitochondrial dysfunction.

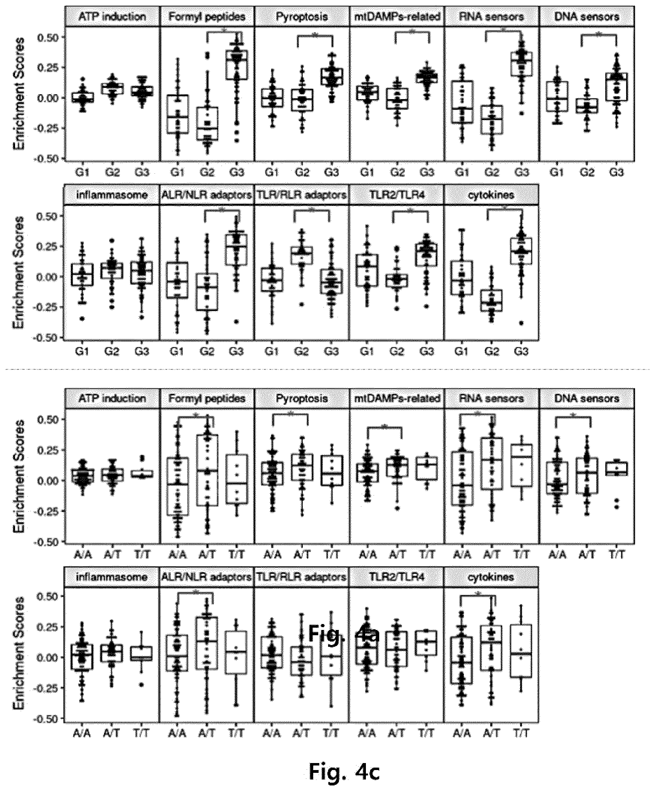

Figure 4C shows the expression patterns of mitochondrial dysfunction-related genes analyzed by class (top) and IFI16 rs6940 genotype (bottom).

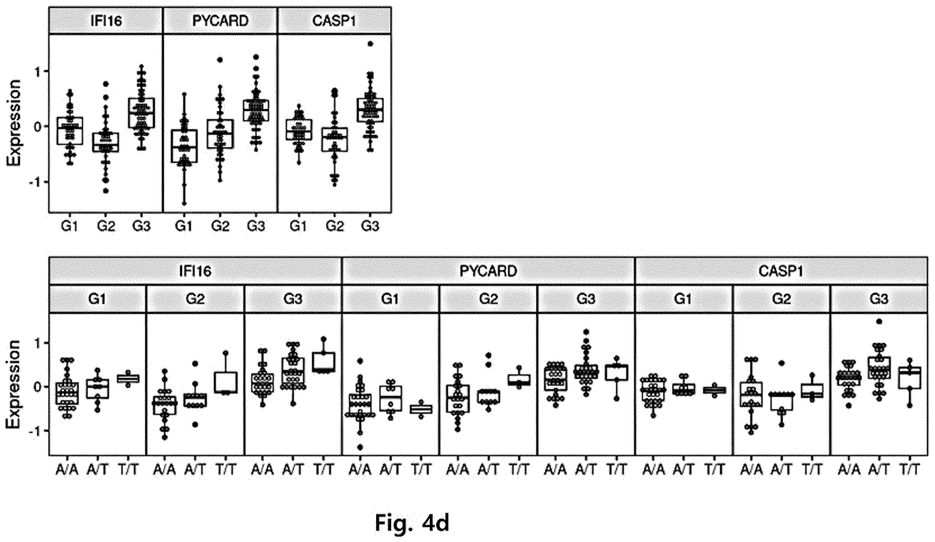

Figure 4D shows IFI16, PYCARD, and CASP1 expression patterns analyzed by class (top) and IFI16 rs6940 genotype (bottom).

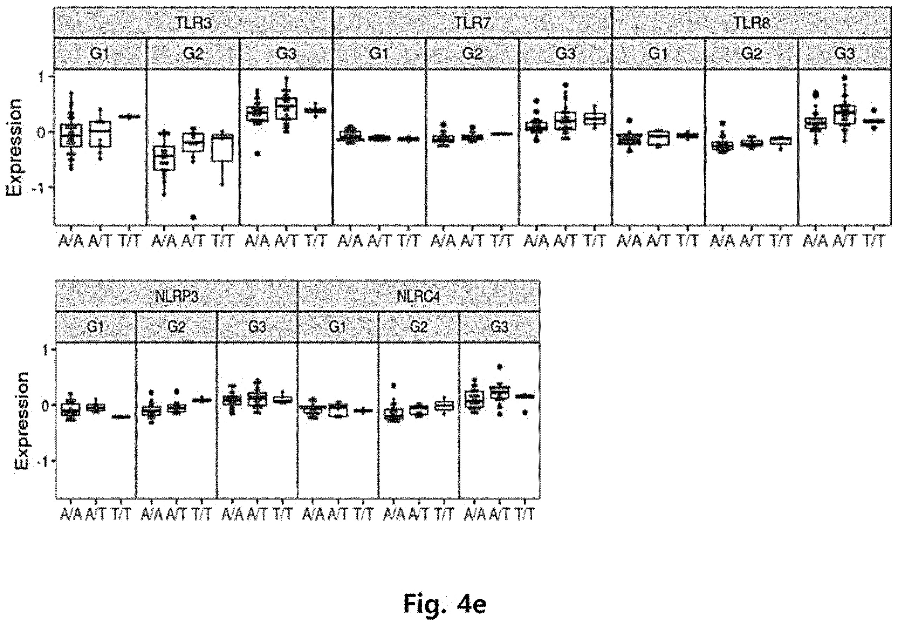

Figure 4E shows the expression patterns of mtDAMP (NLRP3 and NLRC4) and mtRNA (TLR3, TLR7, and TLR8) related genes analyzed by class and IFI16 rs6940 genotype.

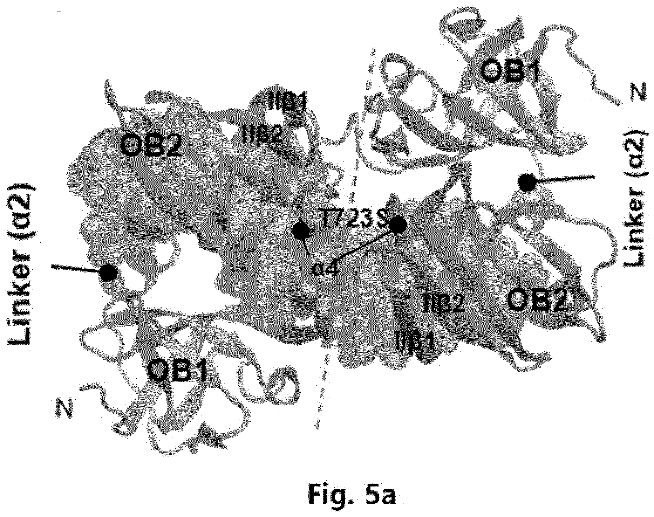

Figure 5A is a schematic diagram of the structural modeling of the IFI16 protein.

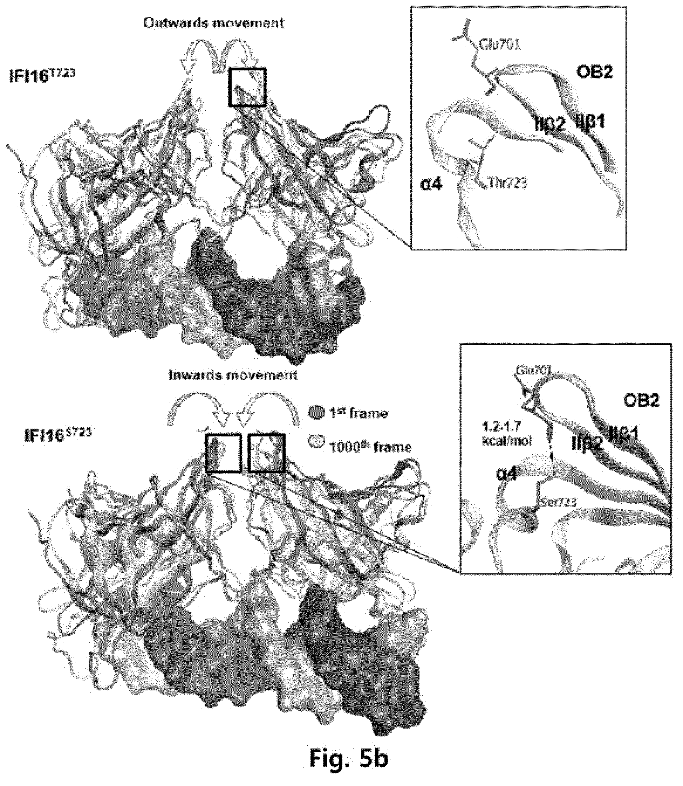

Figure 5B shows data from a molecular dynamics simulation that monitors the conformational changes of two HINb domains bound to dsDNA as a function of time to demonstrate how the variant IFI16^{S723} affects the overall stability of the HINb-DNA binding.

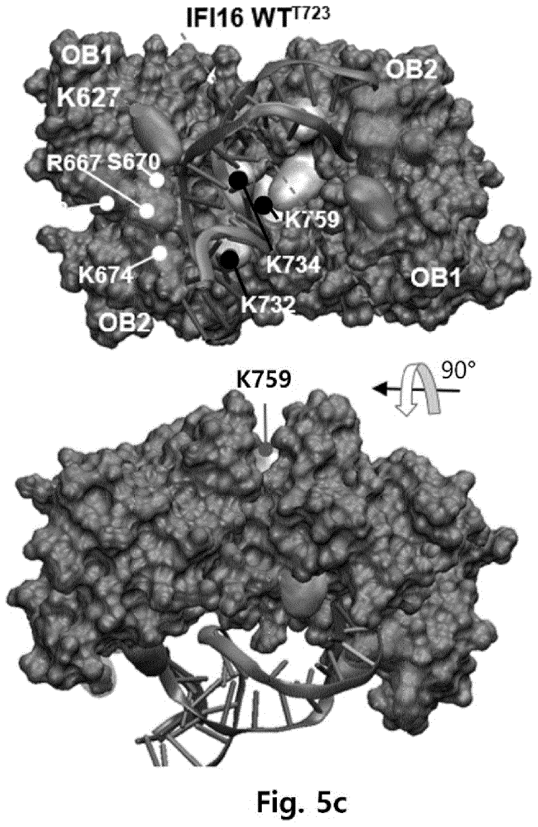

Figure 5C shows structural modeling of how the unstable OB2 domain in the HINb of wild-type IFI16^{T723} breaks the critical salt bridge between L732 and L759 with dsDNA.

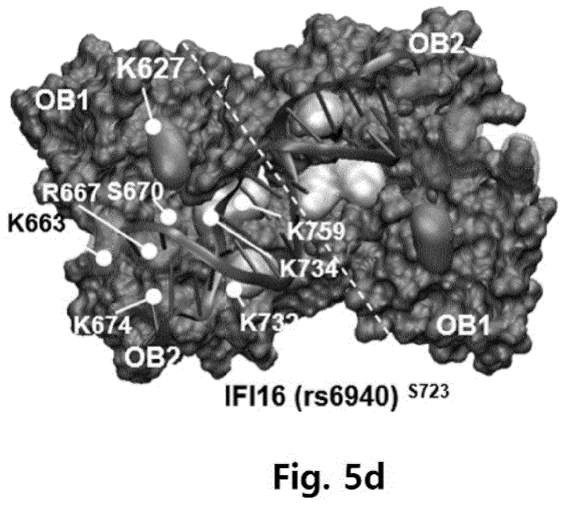

Figure 5D is a schematic diagram of the structural modeling of the variant IFI16^{S723} in the salt bridge retention state.

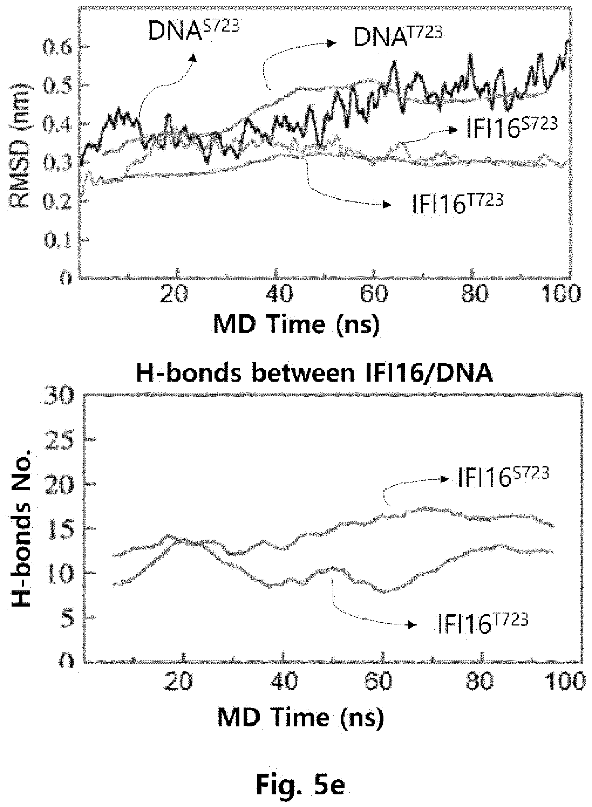

Figure 5E shows the RNSD scores and the number of hydrogen bonds to analyze the stability of the HINb^{S723}-dsDNA and HINb^{T723}-dsDNA bonds.

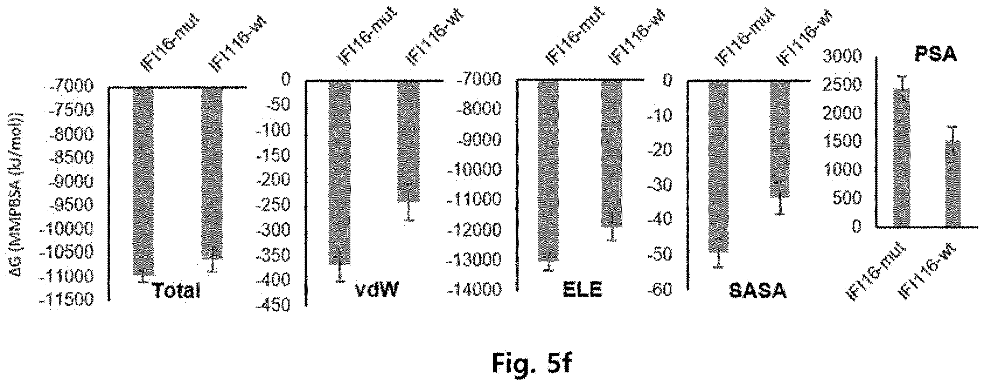

Figure 5F shows the van der Waals (vdW), electrostatic energy, and total DNA binding energy of IFI16^{S723} and IFI16^{T723} by performing a binding free energy perturbation analysis.

### [Mode of the Invention]

The present invention will now be described in detail.

### Biomarker composition for predicting, diagnosing, or prognosticating risk or severity of chronic liver disease

In one aspect, the present invention relates to a biomarker composition for predicting, diagnosing, or prognosticating risk or severity of chronic liver disease, comprising an IFI16 (Interferon Gamma Inducible Protein 16) mutant gene or an IFI16 mutant protein.

The term "prediction of risk or severity" used in the present invention can be interpreted to mean predicting or diagnosing whether there is a possibility of progression of chronic liver disease, whether the likelihood of developing chronic liver disease is relatively high, or whether chronic liver disease has already progressed.

The term "diagnosis" used in the present invention can be interpreted to mean the identification of the presence or characterization of a pathological condition. For the purposes of the present invention, prediction or diagnosis can be the determination of the presence or probable progression of chronic liver disease, in particular non-alcoholic fatty liver disease (NAFLD) or non-alcoholic steatohepatitis (NASH).

The term "prognosis" used in the present invention can be interpreted to mean predicting the course and outcome of chronic liver disease in advance, and more specifically, prognostic prediction may depend on the physiologic or environmental conditions of the patient, and can be interpreted to mean any act of predicting the course and outcome of the disease based on a combination of these patient conditions.

The term "diagnostic biomarker" used in the present invention includes any organic biomolecule, such as a polypeptide or nucleic acid (e.g., mRNA, etc.), lipid, glycolipid, glycoprotein, sugar (monosaccharide, disaccharide, oligosaccharide, etc.), or the like, that exhibits a significant increase or decrease in a particular gene expression level or protein expression level in an individual with chronic liver disease compared to a normal control, and preferably includes the IFI16 mutant gene.

The term "mutant" used in the present invention includes nucleotide and amino acid sequences of a gene that have been base substituted, deleted, inserted, amplified, and rearranged, and a nucleotide variant refers to a change in a nucleotide sequence (e.g., insertion, deletion, inversion, or substitution of one or more nucleotides) relative to a reference sequence (e.g., a wild-type sequence). Preferably this refers to a single nucleotide polymorphism (SNP) or single-nucleotide variant (SNV), and includes a protein that has been mutated thereby.

In the present invention, the IFI16 mutant gene may be one or more single-nucleotide variants (SNVs) selected from the group consisting of rs2276404, rs73021847, rs7532207, and rs6940 of the IFI16 gene, and the IFI16 mutant protein may be a missense variant (T723S) in which a threonine is replaced by a serine at position 723 of the amino acid sequence consisting of SEQ ID NO: 14.

Detection or increased expression of the present IFI16 mutant gene or IFI16 mutant protein can indicate a high likelihood of progression to chronic liver disease, or already having chronic liver disease, and a poor prognosis for chronic liver disease.

In the present invention, the chronic liver disease may be non-alcoholic fatty liver disease (NAFLD) or non-alcoholic steatohepatitis (NASH).

In a specific embodiment of the present invention, genes were screened by RNA expression pattern analysis, whole exome sequencing (WES) and whole genome sequencing (WGS) of tissues or peripheral blood mononuclear cells (PBMCs) from a group of NAFLD/NASH patients as shown in the schematic diagram in Figure 1A.

Furthermore, the consensus cluster from the integrated NAFLD transcriptome data (RSEQ) for GSE135251, GSE167523, and NCC-RSEQ was divided into G1 to G3 classes, and subsequent analysis of class-specific differentially expressed genes (DEGs) showed that the rate of progression from NAFLD to NASH and the extent of fibrosis were increased with class (Figure 1B to Figure 1E). Furthermore, increased ECM (ECM receptor interaction) was found in G2 class compared to G1 class, and increased inflammatory response was found in G3 class compared to G2 class, similar to the clinical manifestations of chronic liver disease (Figure 1F).

In other words, the analysis of the IFI16 mutant gene expression pattern of the present invention can predict or diagnose progression to NASH and increased degree of liver fibrosis when classified as G3 class.

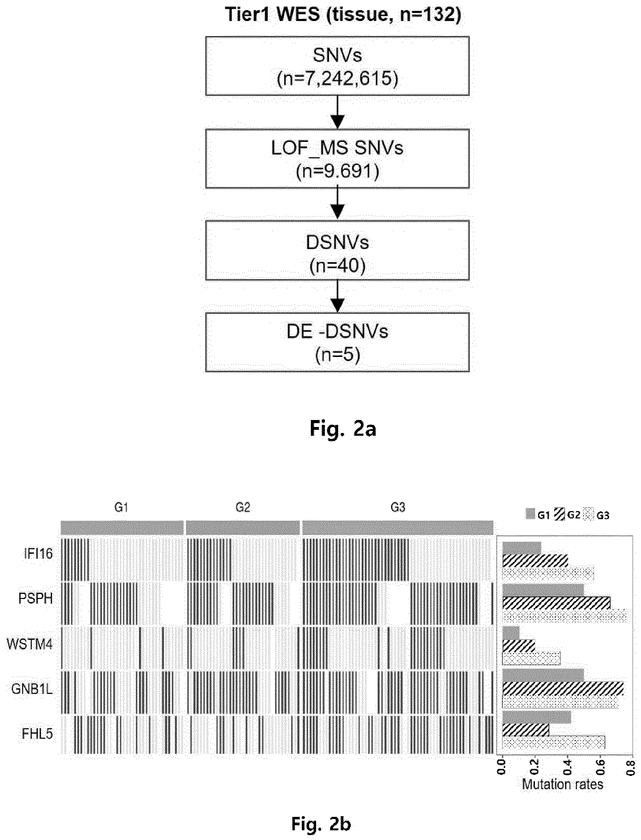

In another specific embodiment of the present invention, five genes with single-nucleotide variants (SNVs) were screened by whole-exome sequencing (WES) of NAFLD patient group tissues using the same method as the schematic diagram in Figure 2A, and the mutation rates of the screened genes were analyzed by class in Figure 1B to finally select the IFI16 gene whose expression was increased with class (Figure 2B).

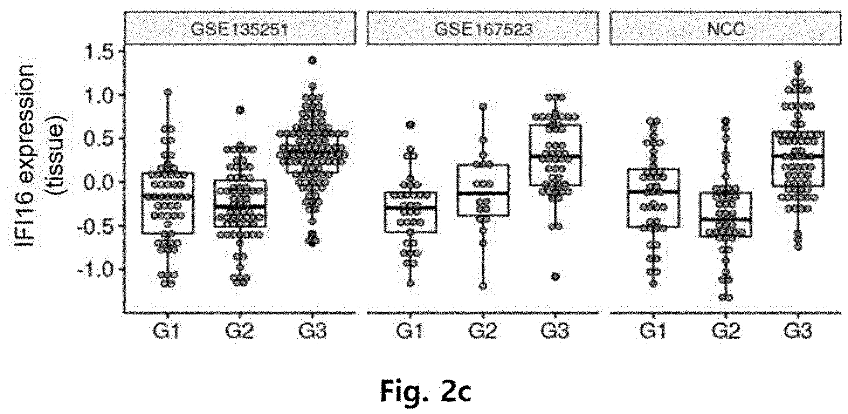

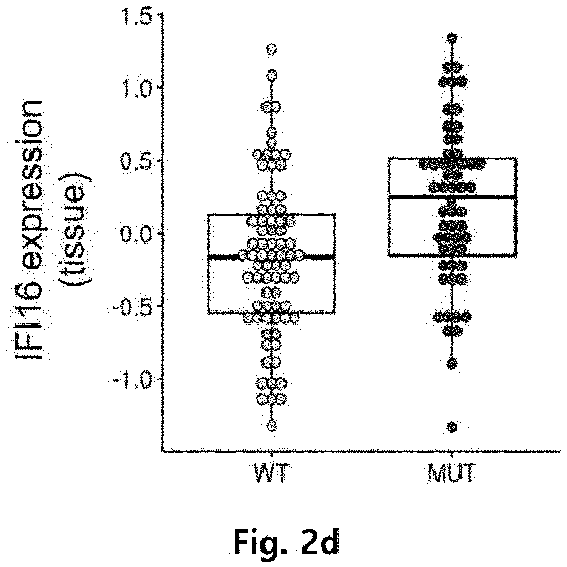

Furthermore, GSE135251, GSE167523, and the National Cancer Center (NCC) NAFLD patient group all showed increased IFI16 gene expression in tissues in G3 class (Figure 2C), and analysis of the expression patterns of the normal IFI16 gene and IFI16 mutant gene in the tissues confirmed an increased IFI16 mutant expression rate (Figure 2D).

In another specific embodiment of the present invention, the expression patterns of IFI16 SNVs, rs2276404, rs73021847, rs7532207, and rs6940 were analyzed, and all four IFI16 SNVs were found to have increased mutation rates and expression specific to G3 class of liver disease (Figure 2E top and Figure 2E bottom). In particular, the frequency of IFI16 rs6940 (A>T) genotype increased stepwise during the progression of G1 to G3 classes (23.7% in G1, 40% in G2, and 55.9% in G3), and IFI16 rs6940 genotype was found to increase stepwise from wild type (A/A), heterozygous (A/T), and homozygous (T/T) (Figure 2F).

In addition, the validation set, RNA expression analysis-whole exome sequencing (RSEQ-WES), confirmed that the expression of the IFI16 gene was increased in accordance with the class stage, and the expression of the IFI16 mutant gene was increased compared to the normal IFI16 gene (Figure 2G). The schematic diagram for IFI16 SNV and the IFI16 SNV mutation rate according to the type of genetic analysis are shown in Figure 2H.

In another specific embodiment of the present invention, NAFLD/NASH-specific cell types were screened by single-cell RNA sequencing (scRNA-Seq) of human hepatocytes using the same method as the schematic diagram in Figure 3A, and the proliferation of each cell type was analyzed by class stage in Figure 1B, and it was found that macrophage proliferation was increased with a class group (Figure 3B and Figure 3E).

We analyzed the correlation of macrophages and gene expression patterns in the pooled RSEQ data (n=460) in Figure 1B, and divided the genes that correlated with macrophage ratio and gene expression into macrophage signatures and non-macrophage signatures to analyze the expression patterns, differentially expressed genes (DEGs) by class in Figure 1B and macrophage-related gene data in Figure 3D were used to categorize differentially expressed genes by class into three types (macrophage signatures (Markers/Non-markers), Mac-independent signatures) (Figure 3B and Figure 3E).

Furthermore, using the pooled RSEQ data (n=460) in Figure 1B, we analyzed the correlation between the IFI16 gene and HPSE gene expression, HPSE gene expression by class, and HPSE gene expression with and without IFI16 rs6940 mutation, and found that HPSE gene was associated with IFI16 gene (mutated) and HPSE gene expression was also increased in G3 class.

In another specific embodiment of the present invention, we confirmed that IFI16 rs6940 expression increases with increased ROS activity (Figure 4A), and IFI16 rs6940 mutants (A/T or T/T) induced downstream expression of mitochondrial dysfunction-related genes, including formyl peptide response, pyroptosis and nucleic acid (NA) sensor response, and worsened mtDNA sensing response through IFI16-PYCARD-CASP1 (Figure 4D through Figure 4E).

In another specific embodiment of the present invention, a comparison of DNA binding of wild-type IFI16^{T723} and variant IFI16^{S723} revealed that the rs6940 variant of IFI16 stabilized the HINb domain, resulting in enhanced binding affinity to dsDNA (Figure 5A to Figure 5E).

These results suggest that when the expression of the rs6940 genotype, an IFI16 SNV, was increased, the HINb domain was stabilized and mitochondrial dysfunction occurred in NAFLD by variant IFI16^{S723} with enhanced binding affinity to dsDNA, and thus the inflammatory response was worsened by immunogenic DNA released from dysfunctional mitochondria.

In other words, the present inventors have confirmed that patients with chronic liver disease can be classified into G1 ~ G3 classes through genetic analysis, and have confirmed that IFI16 mutant gene expression was increased specifically for G1 ~ G3 classes. In particular, the rate of NAFLD and NASH progression and the degree of liver fibrosis was increased in G3 class, thus confirming that the IFI16 mutant gene of the present invention can be used as a biomarker for predicting, diagnosing, or prognosticating risk or severity of chronic liver disease.

Furthermore, we identified that the IFI16 SNV was highly expressed in infiltrating macrophages, playing a critical role in macrophage-induced inflammatory processes, and structural modeling analysis confirmed that the IFI16 variant bound more strongly to dsDNA than wild-type IFI16, exacerbating the impaired mitochondrial DNA-sensing response signaling of the IFI16-PYCARD-CASP1 pathway.

Therefore, the present invention can identify the degree of inflammation and fibrosis of liver disease through mutation analysis of the IFI16 gene, and can provide appropriate treatment methods for chronic liver disease based on the detection of mutations in the IFI16 gene.

### Composition for predicting, diagnosing, or prognosticating risk or severity of chronic liver disease

In another aspect, the present invention relates to a composition for predicting, diagnosing, or prognosticating risk or severity of chronic liver disease, comprising a detection agent for the IFI16 mutant gene or IFI16 mutant protein.

In the present invention, detection or increased expression of the IFI16 mutant gene or IFI16 mutant protein may indicate a high likelihood of progression to chronic liver disease, or may indicate that the patient already has chronic liver disease, and may indicate a poor prognosis for chronic liver disease.

The detection agent for the IFI16 mutant gene may be a primer pair, probe, or antisense nucleotide that specifically binds to the mutant gene, wherein the nucleic acid information of the gene is known in GeneBank or the like, so that one skilled in the art can design the primer pair, probe or antisense nucleotide based on the sequence.

As used herein, the term "primer" refers to a fragment that recognizes a target gene sequence and includes forward and reverse primer pairs, preferably primer pairs that provide assay results with specificity and sensitivity.

As used in the present invention, the term "probe" refers to a substance that can specifically bind to a target substance to be detected in a sample, and through the binding, the presence of the target substance in the sample can be specifically identified. The type of probe may be any substance conventionally used in the art, but is not limited thereto, and may preferably be a peptide nucleic acid (PNA), locked nucleic acid (LNA), peptide, polypeptide, protein, RNA, or DNA, most preferably a PNA.

As used herein, the term "antisense" refers to an oligomer having a sequence of nucleotide bases and an inter-subunit backbone that allows the antisense oligomer to hybridize to a target sequence in RNA by Watson-Crick base-pairing, thereby permitting the formation of an mRNA:RNA:oligomer heterodimer typically within the target sequence. The oligomer may have exact sequence complementarity or approximate sequence complementarity to the target sequence.

In the present invention, IFI16 mutant protein expression levels can also be measured as needed, and for measuring protein expression levels, an antibody, interacting protein, ligand, nanoparticle, or aptamer that specifically binds to a protein or peptide fragment of the IFI16 mutant gene can be used to determine the amount of protein.

The method for measuring or comparing protein expression levels can include protein chip analysis, immunometric, ligand binding assays, matrix desorption/ionization time of flight mass spectrometry (MALDI-TOF) analysis, surface enhanced laser desorption/ionization time of flight mass spectrometry (SELDI-TOF) analysis, radioimmunoassay, radioimmunodiffusion, and Ouchterlony immunodiffusion, rocket immunoelectrophoresis, tissue immunostaining, complement fixation assays, two-dimensional electrophoresis assays, liquid chromatography-mass spectrometry (LC-MS), liquid chromatography-mass spectrometry/mass spectrometry (LC-MS/MS), western blot, and/or enzyme linked immunosorbent assay (ELISA).

### Kit for predicting, diagnosing, or prognosticating risk or severity of chronic liver disease

In another aspect, the present invention relates to a kit for predicting, diagnosing, or prognosticating risk or severity of chronic liver disease, comprising a detection agent for an IFI16 mutant gene or an IFI16 mutant protein.

The kit can be prepared by conventional methods known in the art. The kit may include, for example, an antibody in lyophilized form, buffer, stabilizer, inactive protein, and the like.

The kit may further comprise a detectable label. The term "detectable label" means an atom or molecule that allows specific detection of a molecule containing a label among molecules of the same type without the label. The detectable label may be attached to an antibody, interacting protein, ligand, nanoparticle, or aptamer that specifically binds to the protein or fragment thereof. The detectable label may comprise a radionuclide, a fluorophore, or an enzyme.

The kit may utilize a variety of kits known in the art, and preferably, the kit may be a reverse transcription polymerase chain reaction (RT-PCR) kit or a DNA chip kit.

### Provide information for predicting, diagnosing, or prognosticating risk or severity of chronic liver disease

In another aspect, the present invention relates to a method of providing information for predicting, diagnosing, or prognosticating risk or severity of chronic liver disease, comprising: (a) the step of extracting genomic DNA from a biological sample of the patient; and (b) the step of detecting the IFI16 mutant gene or an IFI16 mutant protein in the extracted genomic DNA.

In the above method, "biological sample" refers to a sample such as tissue, cells, blood, serum, plasma, saliva, cerebrospinal fluid, or urine.

In the method for providing information on the diagnosis of chronic liver disease, the method for detecting the IFI16 mutant gene or IFI16 mutant protein is as described above.

In the present invention, the method of providing information may provide information that detection or increased expression of an IFI16 mutant gene or IFI16 mutant protein is associated with a high risk or severity of progression to chronic liver disease, progression to chronic liver disease, or a poor prognosis for chronic liver disease.

In the present invention, the chronic liver disease can be predicted or diagnosed as non-alcoholic fatty liver disease (NAFLD) or non-alcoholic steatohepatitis (NASH).

In another aspect, the present invention relates to a method of providing information for the treatment of chronic liver disease, comprising: (a) the step of extracting genomic DNA from a biological sample of the patient; and (b) the step of detecting an IFI16 mutant gene or an IFI16 mutant protein in the extracted genomic DNA.

In the present invention, the method of providing the information may provide information about the progress of treatment for chronic liver disease based on the mutation rate of the IFI16 gene or protein.

The present invention will now be described in more detail with reference to the following examples. These embodiments are intended solely to illustrate the invention, and it will be apparent to one of ordinary skill in the art that the scope of the invention is not to be construed as limited by these embodiments.

### Example 1: Selecting genetic groups or patients with chronic liver disease

To screen for markers for the diagnosis of chronic liver disease, two NAFLD/NASH patient gene groups, GSE135251 (n=216) and GSE167523 (n=98), were selected and used for analysis. To compare with a real patient group, NAFLD/NASH patients (n=146) of National Cancer Center (NCC) were selected and their tissues were collected from biopsies or postoperative samples. The combined dataset consisted of liver tissue samples from normal (n=10), NAFL (n=168), and NASH (n=282).

### Example 2: Analyzing gene expression patterns in a group of NAFLD patients

The GSE135251, GSE167523 and NCC patient groups of Example 1 were subjected to RNA expression pattern analysis, whole exome sequencing (WES) and whole genome analysis (WGS) of tissues or peripheral blood mononuclear cells (PBMCs) as shown in the schematic diagram in Figure 1A.

First, subtype classes (G1 to G3) were distinguished via consensus clusters from the integrated NAFLD transcriptome data (RSEQ) for GSE135251, GSE167523, and NCC-RSEQ, and then class-specific differentially expressed genes (DEGs) were screened (permutation t-test) (Figure 1B).

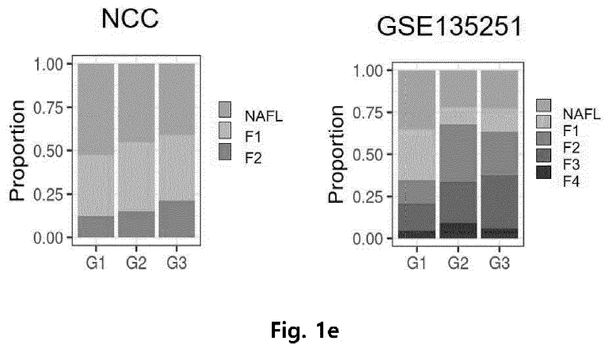

Analysis of the expression patterns distinguished in Figure 1B, divided into G1, G2, and G3 classes, showed that the rate of progression to NASH and the extent of fibrosis were increased with progression from G1 to G3 classes (Figure 1E, Figure 1D, Figure 1F, and Figure 1E).

In addition, Gene set enrichment analysis (GSEA) (MsigDB Hallmark inflammatory response and KEGG ECM gene set) was performed using the classified RNA-seq data (n=460) in Figure 1B, and it was found that ECM (ECM receptor interaction) was increased in G2 class compared to G1 class, and inflammatory response was increased from G2 to G3 class compared to G1 class, confirming the effect of progression of liver disease.

The G1 to G3 classes were significantly associated with patient gender, with a higher proportion of male patients in G1 (76.7%), G2 (74.1%), and G3 (46.9%), and patients with an older average age (> 47 years) were more prevalent in G2/G3 than in G1 (Figure 1D). These results indicate that the class (G1-G3) typing of the present invention well reflects the clinicopathologic features of NAFLD progression independent of the data cohort.

### Example 3: Biomarker screening and expression pattern analysis for predicting or diagnosing chronic liver disease

Genes with single-nucleotide variants (SNVs) were screened by whole-exome sequencing (WES) of NAFLD patient group tissues using the same method as the schematic diagram in Figure 2A.

First, the analysis was performed using WES data (n=132) obtained from patient tissues and analyzed using the following steps.
<Variants call using GATK best practices>
   Step 1: QC (FastQC)
   Step 2: Trimming (Trim_galore)
   Step 3: Alignment (BWA-mem)
   Step 4: Rmdu (Picard)
   Step 5: BQSR (GATK)
   Step 6: Variant call (GATK)
   Step 7: Wild type call (GATK Depth of coverage) (Treated variants with a row depth as Missing)
<Screening>
   Step 1: Screen 7,242,615 SNVs
   Step 2 (LOF_MS SNVs): Functional filter step (Missense SNVs & Loss function SNVs select)
   Step 3 (DSNVs): Class Differential SNVs (fisher p<0.05 & Mutation frequency increase or decrease)
   Step 4 (DE-DSNVs): Determine the difference in expression between the presence and absence of each DSNV (perm.t-test p<0.05 & Fold change > 0.2)

As a result, we selected five genes (DE-DSNVs) that were significantly different between classes, as shown in Figure 2B, and finally selected the IFI16 gene, whose expression was significantly increased by class as the mutation rate of the selected genes increased (Figure 2B, Table 1).

**[Table 1]**

| P-value of selected genes | | |
|---|---|---|
| symbol | avsnp150 | fisher.p |
| IFI16 | rs6940 | 0.004665112 |
| FHL5 | rs2273621 | 0.020326558 |
| PSPH | rs74445297 | 0.024991669 |
| VSTM4 | rs13088 | 0.011662779 |
| GNB1L | rs2073770 | 0.025991336 |

Furthermore, analysis of GSE135251, GSE167523, and National Cancer Center (NCC) RNA-seq (n=460) datasets into the classes in Figure 1B confirmed that IFI16 gene expression in tissues was increased in G3 group in all analyzed groups (Figure 2C, Table 2).

**[Table 2]**

| P-value values by analysis group | |
|---|---|
| Batch | ANOVA p |
| GSE135251 | 1.11 e-15 |
| GSE167523 | 7.79 e-7 |
| NCC | 9.76 e-10 |

Specifically, we identified the expression of IFI16 gene according to the IFI16 rs6940 SNV genotype in tissues using RSEQ and WES (n=132) concordance data in the dataset of NCC, and found that the IFI16 mutant expression rate was increased (p-value: 0.0004399) (Figure 2D).

To improve the accuracy of the analysis, we additionally analyzed NCC PBMC whole genome data (NCC PBMC Whole Genome Seq, n=94) using the same Variants call pipeline and Screening pipeline as NCC_WES, and added ENCODE cCREs (candidate regulatory sequences) and UCSC CpG Island step to the Functional filter step to account for WGS characteristics. This again confirmed that genetic variants of the four DE-DSNVs present in the IFI16 gene, rs2276404 (Promoter), rs73021847 (Enhancer), rs7532207 (Enhancer), and rs6940 (Missense variants), increase by class (top of Figure 2e) (Figure 2E top, Table 3).

**[Table 3]**

| p-value values for IFI16 SNV haplotype analysis results by class | | |
|---|---|---|
| symbol | avsnp150 | fisher.p |
| IFI16 | rs2276404 | 0.018327224 |
| IFI16 | rs73021847 | 0.003665445 |
| IFI16 | rs7532207 | 0.017327557 |
| IFI16 | rs6940 | 0.016327891 |

Furthermore, using the RNA-seq expression values of the four SNVs and matched patients in Figure 2E top, we analyzed the difference in expression values according to the genotype of the four SNVs (WT vs Mut) and found that IFI16 expression was increased in the mutant group in all cases (Figure 2E bottom, Table 4).

**[Table 4]**

| p-value for gene expression difference analysis results by IFI16 SNV genotype | |
|---|---|
| avsnp150 | perm.t p |
| rs2276404 | 0.0001076 |
| rs73021847 | 0.0000437 |
| rs7532207 | 0.002112 |
| rs6940 | 0.002757 |

In particular, as shown in Figure 2F, the frequency of the IFI16 rs6940 (A>T) genotype increased stepwise during the progression of the G1 to G3 classes (23.7% in G1, 40% in G2, and 55.9% in G3), and IFI16 The rs6940 genotype was found to increase stepwise from wild type (A/A), heterozygous (A/T), and homozygous (T/T).

### Example 4: Analyzing IFI16 Gene Expression Patterns Using Validation Set

For data validation, we analyzed RNA-seq data from the validation set using the NTP prediction technique using RNA-seq (n=61) corresponding to Tier 2 in Figure 1.

When analyzed according to the class in Figure 1B, we confirmed that the expression of IFI16 gene was increased in the G3 group in the validation set (Figure 2G, top left), and when analyzing the expression difference according to the IFI16 rs6940 genotype, we confirmed that IFI16 expression was increased in the mutant group compared to the wild-type control (Figure 2G, top right).

Furthermore, in the validation set, as shown in Figure 2F, the IFI16 rs6940 genotype was found to increase stepwise from wild-type (A/A), heterozygous (A/T), and homozygous (T/T) (Figure 2G bottom), indicating that the mutant form of IFI16 rs6940 (A>T) can enhance IFI16 expression and thus promote the progression of NAFLD.

### Example 5: IF116 SNV Analysis

In this invention, the R package (rtracklayer, trackViewer and Gviz) analysis was performed using the Tier1 WES (n=132), Tier1 WGS_BD (n=94) and Tier2 WES (n=61) data in Figure 1, and the IFI16 Lolipop plot using the gene (IFI16-203 / ENST00000359709.7) gene information was shown in Figure 2H. According to the location information, one SNV was a missense mutation causing loss of function, and three were located in promoter and enhancer sites that regulated the expression of the gene, further confirming that they can regulate the expression of the gene.

In addition, the sequence information of each IFI16 SNV is shown in Table 5 below, and the primer sequences for Sanger sequencing of IFI16 SNVs are shown in Table 6. In Table 5, the bolded parts are the target sequences amplified by the primers, and the underlined parts are the mutated parts.

**[Table 5]**

| Sequence information of IFI16 SNV | | | | | |
|---|---|---|---|---|---|
| rsID | Position (GRCh38) | Region (dbSNP ) | Regulator y region (ENCOD E Screen) | Sequence | SEQ ID NO: |
| rs2276404 | chr1:15901016 0_**A**>**G** | 5'UTR | Promoter like | **>hg38_dna range=chr1:159009910-159010410 5'pad=2503'pad=250strand=+ repeatMasking=none** | 1 |
| | | | | TTCTCTGGGGCAATAGCAGAATAGGA GCAAGCCAGCACTAGTCAGCTAACTA AGTGACTCAACCAAGGCCTTTTTTCC TTGTTATCTTTGCAGATACTTCATTTT CTTAGCGTTTCTGGAGATTACAACAT CCTGCGGTTCCGTTTCTGGGAACTTT ACTGATTTATCTCCCCCCTCACACAA ATAAGCATTGATTCCTGCATTTCTGAA GATCTCAAGATCTGGACTACTGTTGA AAAAATTTCCAGTGAGGTGAGTAC TGTTCCTGATTTTGTAAATATGATCTT GTTCCTTCCTTGAAGTCCCCAGAATC ACAAGGGGACAATCAGTATTGGTTAT TCAGGGTCATGGGATGATGGGAGTAG GGCTGAGTATTCAGAAAAGTGAAAA CTGAGTTGCTTGATATGAATCCTTCAT TTACTTAGGAAGATAACAGGCATCTT CTATTCCACCACAACTGAGGACTGAA CAAGAGAAAATGCATTTTGACCGTTG CAGATT | |
| rs73021847 | chr1:15901108 4_**T>C** | Intron | Enhancer like | **>hg38_dna range=chr1:159010784-159011384 5'pad=3003'pad=300strand=+ repeatMasking=none** | 2 |
| | | | | CTCTGCCCTCCTGAAAGTTAATGATTT TTTTTTTCCTTGTGGCAAGGTATAGGG GAGTGGAGGGGAAGGCAGTTAGGAA AAGGTTACTATTGTTTACTTTTCAAAT | |
| | | | | TTTTAAAAGATGTTTTCTATAGCCTGG TACAATATTTCATGTGTGCTTAAATGG AATGTGAGATTCTTAAATGTTGTTTTC AGAATTTTATTAGATAAATGTTGTTAA TTACGTTGTTCAAATTTATTATATCATT ACAGATTTTTTCACCTTGTTCATTTAG TAATTGAAGCATAAACTGAAATCTCC TATTATAAAGTTTGCTTTTTTGGCCG GGCACAGAGGTTCATGCCTGTAATCC CAGTACTTTGGGGGAGACCAAGGCG AGCGGATCACTTGACGTCAGGAGTTC CAGACCAGCCTGGCCAGCATGGCGA AACCCTGTCTCTATTAAAAATACAATA ATTAGCCGGGTATGGTCATGTGTGCCT GTAATCCCAGCTACTCAGGAGACTGA GGCAGGAGAATCGCTTGAACCAGGA GGCAGAGGTTGCAGTGAGCCGAGAC TGTGCCACTACACTCCAGCCTGGGTG ACAGAGCAAGGCTCTGTCTCAA | |
| rs7532207 | chr1:15905463 4_**A>T** | Intron | Enhancer like | >**hg38_dna range=chr1:159054384-159054884 5'pad=2503'pad=250strand=+ repeatMasking=none** | 3 |
| | | | | CATGCTCTCAGATTGCTCCAGTTCTC AGGACCAGCAGTCAAACATTTCAAA CCTTCTTTGATAGCAATTGCACCAGG AATACCTTTTGTACTCTCCCCCTTCCT TCTGCCCAATGAAAACCCTCTCCTCA ACTCTTGTCATTGGGTGCACCAGCTC CTTTCTCTCTCCTGTTGTTCCCTGACA TCTCCTGCTCTTTCACTTGCACTCATG | |
| | | | | CTGAGTAGGAGTGAATATCTCATTTC ACGGTCCAAATTAAACAGAGAGGC ATGACTCAAAGGTCAAGAATTTATTA AGGGAGATAGATGAGAGCGAGAAAA GAATCATTTAGAAAGGAATAGGGGAA GAGATATGGTGCAGGGGGAGGAGAT ACAGTGTGATTGAAGGGAGAAATGT AGGATCATCAGCATCTCAACTGGTCT GTCTTTATCTCTTTCTCCTTCAAGGTC ATCAAGACCAGGAAAAACAAGAAAG ACATACTCAATCCTGATTCAAGTATGG AAACTTCAC | |
| rs6940 | chr1:15905487 8_**A>T** | Exon | MS/LOF | >**hg38_dna range=chr1:159054628-159055128 5'pad=2503'pad=250strand=+ repeatMasking=none** | 4 |
| | | | | AATTAAACAGAGAGGCATGACTCAA AGGTCAAGAATTTATTAAGGGAGATA GATGAGAGCGAGAAAAGAATCATTTA GAAAGGAATAGGGGAAGAGATATGG TGCAGGGGGAGGAGATACAGTGTGA TTGAAGGGAGAAATGTAGGATCATCA GCATCTCAACTGGTCTGTCTTTATCTC TTTCTCCTTCAAGGTCATCAAGACCA GGAAAAACAAGAAAGACATACTCAA TCCTGATTCAAGTATGGAAACTTCA CCAGACTTTTTCTTCTAAAATCTGGA TGTCATTGACGATAATGTTTATGGAGA TAAGGTCTAAGTGCCTAAAAAAATGT ACATATACCTGGTTGAAATACAACAC TATACATACACACCACCATATATACTA | |
| | | | | GCTGTTAATCCTATGGAATGGGGTATT GGGAGTGCTTTTTTAATTTTTCATAGT TTTTTTTTAATAAAATGGCATATTTTG CATCTACAACTTCTATAATTTGAAAAA ATAAA | |

**[Table 6]**

| Primers for IFI16 SNV Detection | | | |
|---|---|---|---|
| rsID | Target Sequence (+-10bp) | Sequence (5'->3') | SEQ ID NO: |
| rs2276404 | ATTTCCAGTGAGGTGAGTA CT | F : GGGCAATAGCAGAATAGGAGC | 5 |
| | | R : TCTCTTGTTCAGTCCTCAGTTGT | 6 |
| rs73021847 | TCTCCTATTATAAAGTTTGC T | F : TGGCAAGGTATAGGGGAGTG | 7 |
| | | R : GCTGGAGTGTAGTGGCACAG | 8 |
| rs7532207 | TCCAAATTAAACAGAGAG GCA | F : CATGCTCTCAGATTGCTCCAG | 9 |
| | | R : TTTCCTGGTCTTGATGACCTTG | 10 |
| rs6940 | AAGTATGGAAACTTCACCA GA | F : GCAGGGGGAGGAGATACAG | 11 |
| | | R : CCCATTCCATAGGATTAACAGC | 12 |

### Example 6: Screening cell types specific to chronic liver disease and analyzing gene expression patterns thereof

NAFLD/NASH-specific cell types were screened by single-cell RNA sequencing (scRNA-Seq) of human hepatocytes as shown in the schematic diagram in Figure 3A, and the proliferation of each cell type was analyzed.

First, GSE115469 single cell RNA-seq data (Human normal liver) from the pooled RSEQ data (n=460) of Example 1 (Figure 1B) was analyzed using the MuSiC deconvolution package. (Figure 3B)

We analyzed the cell proliferation by cell type measured above in a boxplot and consensus class by cell type, and found that macrophage proliferation was increased depending on class stage (Figure 3C).

To analyze the correlation of macrophages and gene expression patterns in the pooled RSEQ data (n=460) of Example 1 (Figure 1B), genes that correlated with macrophage ratio and gene expression (MAC_Sig) were divided into macrophage signatures and non-macrophage signatures to analyze the expression patterns, and MAC_Sig consisted of macrophage markers (n=24) and non-macrophage markers (n=37) (Figure 3D).

Using the class-specific differentially expressed genes (DEGs) in Figure 1B and macrophage-associated gene data in Figure 3D, we categorized the class-specific differentially expressed genes into three types: macrophage signatures (Markers/Non-markers), Mac-independent signatures (Figure 3E).

Among the genes with significant changes in expression, the HPSE (Heparanase) gene was found to be highly significantly associated with the expression of IFI16, and its expression was found to change according to the change in class (Figure 3F, Table 7).

**[Table 7]**

| p-value values based on HPSE/IFI16 gene expression analysis | |
|---|---|
| IFI16 and HPSE Correlation | - pooled eset (n=460) |
| (Figure 3F left) | - corr.p: 2.08e⁻³⁶ |
| | - corr.r: 0.54 |
| HPSE Expression by Class | - pooled eset (n=460) |
| (Figure 3F middle) | - ANOVA.p: 2.22 e⁻¹⁶ |
| HPSE expression according to IFI16 rs6940 | - NCC Tissue WES/RSEQ (n=132) |
| (Figure 3F right) | - perm.t p : 0.0002668 |

### Example 7: Confirming the effect of induction of mitochondrial dysfunction by IFI16 SNV in NAFLD

In NAFLD, macrophage infiltration can cause endoplasmic reticulum stress and mitochondrial damage, which can promote hepatic steatosis, inflammation, and hepatocellular injury, as well as excessive production of cytokines and reactive oxygen species (ROS). Consequently, mitochondrial damage by excessive oxidative stress promotes cytoplasmic release of mitochondrial DNA (mtDNA), mitochondrial damage-associated molecular patterns (mtDAMPs), and immunogenic nucleic acid species (Azzimato, Jager, et al. Sci Transl Med, 2020*).*

IFI16 is a DNA sensor that recognizes dsDNA of viral, bacterial, mitochondrial, and nuclear origin that mediates reactive inflammatory signaling, and DNA sensing by IFI16 can be modulated by mitochondrial dysfunction and ROS production in macrophages.

In this study, we evaluated the expression of mitochondrial dysfunction-related genes (n = 91), including ATP induction, pyroptosis, mitochondrial DAMPs (mtDAMPs), inflammasome, nucleic acid (NA) sensors, and cytokines, which were manually collected and categorized into 11 categories based on their signaling pathways and functions.

As a result, increased expression of mitochondrial-related genes and ROS activity were observed during NAFLD progression, as shown in Figure 4A. In particular, the expression of IFI16 rs6940 (A>T), an IFI16 SNV, increased as ROS activity increased, and heterozygous (A/T) and homozygous (T/T) expression increased stepwise during G1 to G3 class progression.

Furthermore, as shown in Figure 4B and Figure 4C, G3 class showed higher expression of genes related to formyl peptide receptor and pyroptosis but lower expression of ATP synthesis compared to G1 and G2 classes, indicating that G3 class was subjected to increased mitochondrial stress compared to G2 class. In particular, G2 class showed lower expression of the formyl peptide response, pyroptosis, mt-DAMP, nucleic acid (NA) sensors, and TLR2/TLR4 compared to G3 class, indicating that G2 class was fighting against elevated oxidative stress.

On the other hand, inflammasome-related genes such as NLRP1, NLRP4, and NLRC4 were not repressed in G2 class compared to G3 class, indicating that these pathways were regulated by general DAMPs rather than mitochondrial stress-related DAMPs. Nucleic acid (NA) sensors were significantly expressed in G3 class, indicating that the mitochondrial membrane was permeable and immunogenic NA species leaked into the cytosol. Overall, these results indicated that IFI16 expression was low in the G2 class but high in the G3 class because mitochondrial stress was low in the G2 class but high in the G3 class.

Furthermore, we analyzed whether downstream signaling was altered by IFI16 SNVs and found that, as shown in Figure 4D, compared to IFI16 rs6940 wild-type A/A, IFI16 mutants (A/T or T/T) induced downstream expression of genes related to mitochondrial dysfunction, including formyl peptide response, pyroptosis, and nucleic acid (NA) sensor response.

IFI16 and AIM2 induced IFN-I through the IRF3 pathway and CASP1 pathway by directly recruiting the PYCARD adaptor through PYD-PYD domain interactions. As shown in Figure 4E, the expression levels of PYCARD and Caspase 1 (CASP1) were higher in the A/T or T/T genotypes than in the IFI16 SNV rs6940 A/A genotype, indicating that the IFI16 SNV was probably adaptive to the IFI16 downstream pathway of PYCARD-CASP1. The PYCARD-CASP1 pathway was influenced by other inflammasomes, including AIM2, NLRP3, and NLRC4, but these were not associated with the G1 to G3 class or the IFI16 SNP.

In addition to mtDNA, mitochondrial dysfunction led to leakage of mtDAMPs and mtRNA, which were detected by NLRP1/3-NLRC4 and TLR/RLR, respectively, but not by IFI16. As expected, the expression of these sensors for mtDAMPs (e.g., NLRP1, NLRP3, and NLRC4) and mtRNAs (e.g., TLR3, TLR7, and TLR8) was not associated with their G1 to G3 class or IFI16 SNVs (Figure 4E).

In other words, we confirmed that the IFI16 SNV rs6940 (A/T or T/T) of the present invention can exacerbate the mtDNA sensing response via IFI16-PYCARD-CASP1, but does not exacerbate the mtDAMP or mtRNA sensing response during NAFLD progression.

### Example 8: Analyzing the structural conformation of the IFI16 SNV and identifing DNA detection reaction

IFI16 SNV rs6940 is a missense variant (T723S) that replaces a threonine with a serine, so the structural change is expected to alter IFI16-DNA binding affinity.

To compare the DNA binding of wild-type IFI16^{T723} (SEQ ID NO: 13; UniProtKB/Swiss-Prot: Q16666.3) and variant IFI16^{S723} (SEQ ID NO: 14), a structural modeling analysis was performed using IFI16 protein structure data from RSCB-PDB.

The IFI16 protein contains two DNA-binding HINa and HINb domains and one PYRIN domain, and the T723S variant is located in the HINb domain that recognizes DNA (Tengchuan Jin et. al., Immunity, 36(4):561-571, 2012). Based on crystallographic studies, the IFI16 HINb-dsDNA interface was established through electrostatic interactions between the negatively charged sugar-phosphate backbone and positively charged residues. The N-terminus of the HINb domain was located away from the DNA binding interface, potentially facilitating the interaction of the PYRIN domain with other PYRIN domains containing adaptors such as PYCARD for further downstream processing such as caspase-1 activation.

As shown in Figure 5A, the HINb domain of IFI16 contained typical oligonucleotide binding 1 (OB1) and OB2 folds connected via a linker helix (α2), and structural modeling confirmed that IFI16 bound to dsDNA by establishing a salt bridge between the positively charged residues of OB1, linker helix α2, and OB2 domains and the backbone phosphate group of DNA.

To demonstrate how the variant IFI16^{S723} affects the overall stability of the HINb-DNA binding, molecular dynamics simulations were performed to monitor the conformational changes of the two HINb domains bound to dsDNA as a function of time. As shown in Figure 5B, the OH group of S723 in IFI16^{S723} formed a strong hydrogen bond (l = 1.7 Å & E = 1.2-1.7 kcal/mol) with the backbone oxygen (O) of G701, which was absent in IFI16^{T723}. G701 was located in the hinge loop between llβ and llβ in the OB2 domain.

Furthermore, interface analysis revealed that the unstable OB2 domain in the HINb of wild-type IFI16^{T723} broke the critical salt bridge between L732 and L759 with dsDNA (Figure 5C), whereas the mutant IFI16^{S723} kept the salt bridge intact (Figure 5D). This may explain where the linker helix and OB1, but not OB2, play an important role in strong binding AIM2 to dsDNA (Kd = 0.034 µM).

Furthermore, the stability of the HINb^{S723} -dsDNA binding could be supported by the smooth conformational behavior of the root-mean-square-deviation (RMSD) and root-mean-square-fluctuation (RMSF) scores, whereas the binding between HINb^{T723} and dsDNA showed strict changes in these scores (Figure 5E left). We demonstrated that the van der Waals (vdW) forces and the number of hydrogen bonds remained stable in the HINbS723-dsDNA binding than in the HINb T723-dsDNA (Figure 5E right).

Furthermore, binding free energy perturbation analysis using the Poisson-Boltzmann Surface Area (MM-PBSA) implemented in GROMACS v5.0 demonstrated that IFI16^{S723} had lower van der Waals (vdW) and electrostatic energies than IFI16^{T723}, as shown in Figure 5F. The overall DNA binding energy of IFI16^{T723} (10,616.73 kJ/mol) was also found to be significantly lower than that of IFI16^{S723} (-10,978.48 kJ/mol).

In other words, the above results suggest that the rs6940 variant of IFI16 of the present invention stabilizes the HINb domain, enhancing its binding affinity for dsDNA, and exacerbates the inflammatory response caused by immunogenic DNA released during mitochondrial dysfunction in advanced NAFLD.

In the present invention, as a result of performing genomic analysis on NAFLD and NASH patient groups, it was confirmed that the frequency of IFI16 single-nucleotide variants (SNVs) including rs2276404, rs73021847, rs7532207, and rs6940 was increased, and the expression of the IFI16 mutant gene was increased depending on the disease stage of liver disease. Furthermore, we confirmed that IFI16 SNVs induced macrophage-induced inflammatory processes and exacerbated mitochondrial DNA-sensing response signaling. Thus, the IFI16 mutant gene of the present invention can be useful for predicting, diagnosing, or prognosticating risk or severity of chronic liver disease.

## Claims

1. A biomarker composition for predicting, diagnosing, or prognosticating risk or severity of chronic liver disease, comprising an Interferon Gamma Inducible Protein 16 (IFI16) mutant gene or an IFI16 mutant protein.

2. The biomarker composition for predicting, diagnosing, or prognosticating risk or severity of chronic liver disease according to claim 1, wherein the IFI16 mutant gene is one or more single-nucleotide variants (SNVs) selected from the group consisting of rs2276404, rs73021847, rs7532207, and rs6940 of the IFI16 gene.

3. The biomarker composition for predicting, diagnosing, or prognosticating risk or severity of chronic liver disease according to claim 1, wherein the IFI16 mutant protein is a missense variant (T723S) in which a threonine is replaced by a serine at position 723 of the amino acid sequence consisting of SEQ ID NO: 14.

4. The biomarker composition for predicting, diagnosing, or prognosticating risk or severity of chronic liver disease according to claim 1, wherein the chronic liver disease is non-alcoholic fatty liver disease (NAFLD) or non-alcoholic steatohepatitis (NASH).

5. A composition for predicting, diagnosing, or prognosticating risk or severity of chronic liver disease, comprising a detection agent for an Interferon Gamma Inducible Protein 16 (IFI16) mutant gene or IFI16 mutant protein.

6. The composition for predicting, diagnosing, or prognosticating risk or severity of chronic liver disease according to claim 5, wherein the IFI16 mutant gene is one or more single-nucleotide variants (SNVs) selected from the group consisting of rs2276404, rs73021847, rs7532207, and rs6940 of the IFI16 gene.

7. The composition for predicting, diagnosing, or prognosticating risk or severity of chronic liver disease according to claim 5, wherein the IFI16 mutant protein is a missense variant (T723S) in which a threonine is replaced by a serine at position 723 of the amino acid sequence consisting of SEQ ID NO: 14.

8. The composition for predicting, diagnosing, or prognosticating risk or severity of chronic liver disease according to claim 5, wherein the chronic liver disease is non-alcoholic fatty liver disease (NAFLD) or non-alcoholic steatohepatitis (NASH).

9. The composition for predicting, diagnosing, or prognosticating risk or severity of chronic liver disease according to claim 5, the detection agent is a primer pair, probe, or antisense nucleotide that specifically binds to the mutant gene, or an antibody, interacting protein, ligand, nanoparticle, or aptamer that specifically binds to a mutant protein.

10. A kit for predicting, diagnosing, or prognosticating risk or severity of chronic liver disease, comprising the composition for predicting, diagnosing, or prognosticating risk or severity of chronic liver disease of any one of claims 5 to 9.

11. A method of providing information for predicting, diagnosing, or prognosticating risk or severity of chronic liver disease, comprising:
(a) the step of extracting genomic DNA from a biological sample of the patient; and
(b) the step of measuring the detection or expression level of an IFI16 mutant gene or an IFI16 mutant protein in the extracted genomic DNA.

12. The method of providing information for predicting, diagnosing, or prognosticating risk or severity of chronic liver disease according to claim 11, wherein the IFI16 mutant gene is one or more single-nucleotide variants (SNVs) selected from the group consisting of rs2276404, rs73021847, rs7532207, and rs6940 of the IFI16 gene.

13. The method of providing information for predicting, diagnosing, or prognosticating risk or severity of chronic liver disease according to claim 11, wherein the IFI16 mutant protein is a missense variant (T723 S) in which a threonine is replaced by a serine at position 723 of the amino acid sequence consisting of SEQ ID NO: 14.

14. The method of providing information for predicting, diagnosing, or prognosticating risk or severity of chronic liver disease according to claim 11, wherein the method provides information that there is a high risk or severity of progression to chronic liver disease when the IFI16 mutant gene or IFI16 mutant protein is detected or increased in expression.

15. The method of providing information for predicting, diagnosing, or prognosticating risk or severity of chronic liver disease according to claim 11, wherein the method provides information that chronic liver disease occurs when the IFI16 mutant gene or IFI16 mutant protein is detected or increases in expression.

16. The method of providing information for predicting, diagnosing, or prognosticating risk or severity of chronic liver disease according to claim 11, wherein the method provides information that the prognosis for chronic liver disease is poor when the IFI16 mutant gene or IFI16 mutant protein is detected or increased in expression.

17. The method of providing information for predicting, diagnosing, or prognosticating risk or severity of chronic liver disease according to claim 11, wherein the chronic liver disease is non-alcoholic fatty liver disease (NAFLD) or non-alcoholic steatohepatitis (NASH).
